(19) Europäisches Patentamt
European Patent Office
Office européen des brevets

(11) Veröffentlichungsnummer : **0 177 807**
**B1**

(12) # EUROPÄISCHE PATENTSCHRIFT

(45) Veröffentlichungstag der Patentschrift : **15.03.89**

(21) Anmeldenummer : **85111671.5**

(22) Anmeldetag : **16.09.85**

(51) Int. Cl.⁴ : **C 07 C 69/24**, C 07 C 69/06, C 07 C 69/14, C 11 B 9/00, A 61 K 7/46, A 23 L 1/226, C 07 C 69/675

(54) **Hexanoate, Verfahren zu deren Herstellung und Riech- und/oder Geschmackstoffkompositionen mit einem Gehalt an solchen Verbindungen.**

(30) Priorität : **10.10.84 CH 4856/84**
**06.08.85 CH 3370/85**

(43) Veröffentlichungstag der Anmeldung : **16.04.86 Patentblatt 86/16**

(45) Bekanntmachung des Hinweises auf die Patenterteilung : **15.03.89 Patentblatt 89/11**

(84) Benannte Vertragsstaaten : **CH DE FR GB LI NL**

(56) Entgegenhaltungen :
**FR--A-- 2 339 396**
**US--A-- 3 852 307**
**INDIAN JOURNAL OF CHEMISTRY, Band 16B, März 1978, Seiten 188-190, New Delhi, Indien; J.S. PATEL et al.: "Synthetic studies of roseoxide"**
**INDIAN JOURNAL OF CHEMISTRY, Band 23B, Oktober 1984, New Delhi, Indien; R.S. RANDAD et al.: "Synthesis of m-phenoxybenzyl 6-halo/cyano/alkyl-3-methyl-5-enoates from (+)-citronellol"**

(73) Patentinhaber : **L. GIVAUDAN & CIE Société Anonyme**
**CH-1214 Vernier-Genève (CH)**

(72) Erfinder : **Ochsner, Paul Albert, Dr.**
**30 Avenue des Tilleuls**
**CH-1203 Genf (CH)**

(74) Vertreter : **Urech, Peter, Dr. et al**
**Grenzacherstrasse 124 Postfach 601**
**CH-4002 Basel (CH)**

**EP 0 177 807 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Die Erfindung betrifft neue Riechstoffe. Es handelt sich dabei um die Verbindungen der Formel

$$R^2COO—(CH_2)_5—COOR^1 \qquad (I)$$

worin $R^1$ $C_{1-4}$-Alkyl oder $C_{2-4}$-Alkenyl und $R^2$ Wasserstoff, $C_{1-3}$-Alkyl, $C_{2-3}$-Alkenyl oder $C_{1-2}$-Alkoxy bedeuten.

Die Alkyl- und Alkenylreste können geradkettig oder verzweigt sein. Beispiele solcher Reste sind : Methyl, Aethyl, Propyl, Isopropyl, Butyl, Isobutyl, Vinyl, Allyl, Propenyl, Methallyl, Methoxy, Aethoxy, etc.

Die Formel I soll im übrigen sämtliche im Hinblick auf vorhandene cis/trans-Isomerie möglichen geometrischen Isomeren erfassen.

Die Verbindungen I weisen besondere organoleptische Eigenschaften auf, auf Grund derer sie sich vorzüglich als Riechstoffe eignen.

Die Erfindung betrifft demgemäss auch die Verwendung der Verbindungen I als Riechstoffe und Riechstoffkompositionen mit einem Gehalt an Verbindungen I.

Bevorzugt sind das 6-Acetoxy-äthyl-hexanoat, das 6-Isobutyroxy-äthyl-hexanoat und das 6-Propionoxy-allyl-hexanoat.

Weitere interessante Ester sind : 6-Formoxy-methyl-hexanoat, 6-Formoxy-allyl-hexanoat, 6-Formoxyisobutyl-hexanoat, 6-Acetoxy-allyl-hexanoat und 6-Acetoxy-isobutyl-hexanoat, 6-Aethoxycarbonyloxy-äthyl-hexanoat.

Die Erfindung betrifft auch ein Verfahren zur Herstellung der Verbindungen I. Dieses Verfahren ist dadurch gekennzeichnet, dass man eine Verbindung der Formel

$$HO—(CH_2)_5—COOR^1 \qquad (II)$$

worin $R^1$ obige Bedeutung hat, verestert.

Die Veresterung der Alkohole II wird in an sich bekannter Weise unter Verwendung der üblichen Acylierungsmittel, also z. B. Acylhalogeniden oder Säureanhydriden durchgeführt. Bevorzugt ist die Arbeitsweise unter Verwendung der Säureanhydride. Man arbeitet zweckmässigerweise in Anwesenheit von Phosphorsäure.

Man kann aber auch, wie gesagt, unter Verwendung der Acylhalogenide arbeiten ; in diesem Fall wird zweckmässigerweise in Anwesenheit von tertiären Aminen, wie Pyridin oder N,N-Dimethylanilin gearbeitet.

Die Kohlensäureester I ($R^2 = C_{1-2}$-Alkoxy) werden in an sich bekannter Weise hergestellt, also zweckmässigerweise durch Umsetzung der Alkohole II mit Chlorameisensäureester in Anwesenheit von tertiären Aminen, wie Pyridin oder N,N-Dimethylanilin.

Die Ester I werden zweckmässig durch Destillation unter vermindertem Druck gereinigt ; sie stellen farblose bis leicht gelblich gefärbte Flüssigkeiten dar, sind unlöslich in Wasser, aber löslich in organischen Lösungsmitteln, wie z. B. Alkoholen, Aethern, Ketonen, Estern, Kohlenwasserstoffen und halogenierten Kohlenwasserstoffen.

Die Verbindungen der allgemeinen Formel I weisen, wie oben gesagt, besondere organoleptische Eigenschaften auf.

Die Verbindungen der Formel I zeichnen sich insbesondere durch eine Kombination von fruchtigen Noten und ausgesprochener Haftfestigkeit aus. Sie verstärken auch Holz- und Moschusnoten von Riechstoffkompositionen, ohne dass dabei ihr Eigengeruch störend wirkt.

Im Vordergrund des Interesses stehen die Himbeernoten, wie sie insbesondere das 6-Acetoxy-äthyl-hexanoat und das 6-Isobutyroxy-äthyl-hexanoat in ausgesprochener Weise aufweisen.

Es sind zwar bereits eine Reihe von Himbeerriechstoffen bekannt, z. B. das Piperonylaceton oder das p-Hydroxybenzylaceton. Es handelt sich jedoch dabei durchweg um Verbindungen, die aufgrund ihrer Struktur um ein Vielfaches teurer sind.

Ueberraschend im Falle von 6-Acetoxy-äthyl-hexanoat und von 6-Aethoxycarbonyloxy-äthyl-hexanoat ist nun die Verstärkung der Holznote des Himbeeraromas in konventionellen Himbeerkompositionen und das Auftreten eines ausgesprochen fixativen Effekts, ohne dass dabei die fruchtige Note in irgend einer Weise negativ beeinflusst würde.

Auch die übrigen Verbindungen I zeichnen sich insbesondere durch fruchtige Noten aus, wobei aber in gewissen Fällen auch noch blumige Nebennoten erwähnenswert sind.

Erwähnenswert sind hier insbesondere noch das 6-Propionoxy-methyl-hexanoat (Narzisse) und das 6-Acetoxy-isobutyl-hexanoat (nach Nerolidol).

Die Verbindungen der Formel I eignen sich auf Grund ihrer Geruchsnoten insbesondere beim Verstärken von fruch — tigen Noten und als Verstärker von Moschusnoten. Daneben eigenen sie sich zur Modifizierung einer ausgesprochen breiten Palette von bekannten, z. B.

a) blumigen Kompositionen, in denen z. B. die warmen Moschusnoten verstärkt zum Ausdruck kommen sollen (z. B. für Herren-Colognes),

b) des weiteren aber auch von Chypre-Kompositionen (Extrait-Typen, Kompositionen der femininen Richtung), von

c) Tabak- und Holz- und Fougère-Kompositionen, (Extrait-Typen der masculinen Richtung) und von

d) Kompositionen mit grünen Noten, wo insbesondere eine erwünschte Verstärkung und Abrundung und harmonisierende Effekte erzielt werden.

Als Riechstoffe eignen sich die Verbindungen I auf Grund ihrer oben beschriebenen, originellen Noten, insbesondere in Kombination mit einer Reihe von natürlichen und synthetischen Riechstoffen, wie z. B.

Naturprodukten
wie Angelikawurzöl, Galbanumöl, Vetiveröl, Patchouliöl, Sandelholzöl, Mandarinöl, Muskatellersalbei, Ylang-Ylang-öl, Cedernöl, Fichtenöl, Lavendelöl, Bergamotteöl, Citronenöl, Orangenöl, Corianderöl, Eichenmoos, Castoreum, Ciste labdanum, Calmusöl, Geraniumöl, Jasmin absolue, Rosenöl, Cassis absolue, Narzissen absolue, Verveine absolue, Grapefruit-Extrakten, usw.

Aldehyden
wie $C_{10}$-, $C_{11}$-, $C_{14}$-Aldehyd, Hydroxycitronellal, Cyklamenaldehyd, Benzaldehyd, p-tert.-Butyl-α-methyl-hydrozimtaldehyd, Citral, Citronellal, 2,6-Dimethyl-5-hepten-1-al, Isovaleraldehyd, trans-2-Hexenal, trans-2-Octenal, n-Octanal, n-Nonanal, trans-2-cis-6-Nonadienal, 2,4-Decadienal, Methylnonyl-acetaldehyd, Cyclal C (1,3-Dimethyl-cyclohex-1-en 4 (und 5)-carboxaldehyd), Lyral, usw.

Ketonen
wie alpha-Jonon, beta-Jonon, Methyljonon, Allyljonon, Acetanisol, 4-(para-Hydroxyphenyl)-2-butanon, Campher, Menthon, Carvon, Pulegon, p-Methylacetophenon, Methylamylketon, usw.

Acetalen und Ketalen
wie Phenylacetaldehyd-dimethylacetal, Phenylacetaldehyd-glycerinacetal, 2-Methyl-1,3-dioxolan-2-äthylacetat, Capronaldehyd-dimethylacetal, Acetal R (gemischtes Acetal von Acetaldehyd mit Phenyläthylalkohol und n-Propanol), usw.

Aethern
wie Eugenolmethyläther, Methyl-1-methyl-cyclododecyläther, Anethol, Estragol, Rosantolène (Methyläthylsaligenin), usw.

Phenolkörpern
wie Vanillin, Eugenol, Creosol, Chavicol, usw.

Alkoholen
wie Butanol, n-Hexanol, cis-3-Hexenol, trans-2-cis-6-Nonadienol, cis-6-Nonenol, Linalool, Geraniol, Rhodinol, Nerol, Citronellol, Nerolidol, Farnesol, Benzylalkohol, Phenyläthylalkohol, Zimtalkohol, Terpineol, Patchone (4-tert.-Butylcyclohexanol), usw.

Estern
wie Aethylformiat, Aethylacetat, Isoamylacetat, t-Butylcyclohexylacetat, Myraldylacetat$^R$ (Givaudan), Benzylacetat, Styrallylacetat, Aethyl-α-methyl-phenylglycidat, Maltylisobutyrat, Dimethylbenzylcarbinylacetat und butyrat, Linalylacetat, Isobutylacetat, n-Amylbutyrat, n-Amylvalerianat, Aethylpalmitat, Cinnamylformiat, Terpenylacetat, Geranylacetat, Hexylsalicylat, Linalylanthranilat, Amylsalicylat, Methyldihydrojasmonat, Benzylsalicylat.

Lactone
wie γ-Undecalacton, γ-Decalacton, γ-Nonalacton, δ-Decalacton, δ-Octalacton, Cumarin, usw.

Säuren
wie Geranylsäure, Citronellylsäure, Zimtsäure, Phenylessigsäure, usw.

schwefelhaltigen Verbindungen
wie p-Menthan-8-thiol-3-on, Dimethylsulfid und anderen Sulfiden und Disulfiden, usw.

stickstoffhaltigen Verbindungen
wie Methylanthranilat, Indol, Isobutylchinolin, verschiedenen Pyrazinen, 5-Methyl-heptan-3-on-oxim, Nitromoschus, usw.

verschiedenen weiteren in der Parfümerie oft benutzten Komponenten
wie Keton-Moschus, macrocyclische Moschuskörper wie Musk 174[R] (12-Oxahexadecanolid), Sandela (Isocamphylcyclohexanol), polycyclische Moschuskörper wie Fixolid, Galaxolid.

Die Verbindungen der Formel I lassen sich in weiten Grenzen einsetzen, die beispielsweise von 0,1 (Detergentien) — 50 % (alkoholische Lösungen) in Kompositionen reichen können, ohne dass diese Werte jedoch Grenzwerte darstellen sollen, da der erfahrene Parfümeur auch mit noch geringeren Konzentrationen Effekte erzielen oder aber mit noch höheren Dosierungen neuartige Komplexe aufbauen kann. Die bevorzugten Konzentrationen bewegen sich zwischen 0,5 und 20 %. Die mit I hergestellten Kompositionen lassen sich für alle Arten von parfümierten Verbrauchsgütern einsetzen (Eaux de Cologne, Eaux de Toilette, Extraits, Lotionen, Crèmes, Shampoos, Seifen, Salben, Puder, Desodorantien, Detergentien, Raumparfums, etc.).

Die Verbindungen I können demgemäss bei der Herstellung von Kompositionen und — wie obige Zusammenstellung zeigt — unter Verwendung einer breiten Palette bekannter Riechstoffe verwendet werden. Bei der Herstellung solcher Kompositionen können die oben aufgeführten bekannten Riechstoffe nach (dem Parfümeur bekannter) Art und Weise verwendet werden, wie z. B. aus W. A. Poucher, Perfumes, Cosmetics and Soaps 2, 7. Auflage, Chapman und Hall, London, 1974 hervorgehend.

Beispiel 1

In einem mit Tropftrichter, Thermometer und Rührer versehenen Rundkolben werden 240 g 6-Hydroxy-äthylhexanoat vorgelegt. Innerhalb einer halben Stunde wird unter Rühren ein Gemisch von 180 g Essigsäureanhydrid und 3 g 85 %iger Phosphorsäure zugetropft. Die Temperatur wird durch Kühlen während der Zugabe bei 25 °C gehalten. Danach wird das Reaktionsgemisch langsam auf 50 °C erwärmt und bei dieser Temperatur während 3 Stunden gehalten. Nach dem Abkühlen versetzt man mit Eiswasser und nimmt in 500 ml Hexan auf. Die Hexanlösung wird mit Wasser, mit 100 ml einer 10 %igen Natriumcarbonatlösung und hierauf mit Wasser neutral gewaschen. Nach dem Trocknen über Natriumsulfat wird das Lösungsmittel abdestilliert. Man erhält 282 g Rohprodukt. Die fraktionierte Destillation ergibt 223 g (Ausbeute 73,7 % der Theorie) chemisch reines 6-Acetoxy-äthylhexanoat, davon können 190 g ohne weitere Behandlung als olfaktisch reines Produkt verwendet werden.

Auf analoge Weise werden ausgehend aus den Hydroxyestern $HO(CH_2)_5COOR^1$ (II) weitere Acyloxyhexanoate $R^2COO(CH_2)_5COOR^1$ (I) hergestellt.

Die Hydroxyester $HO(CH_2)_5COOR^1$ (II) sind aus ε-Caprolacton und dem entsprechenden $R^1$-Alkohol $R^1OH$ in Anwesenheit von Natriumbisulfat zugänglich :

| | Sdp [°C/mmHg] | $n_D^{20}$ |
|---|---|---|
| 6-Hydroxy-methylhexanoat | 105° /7 | 1,4385 |
| 6-Hydroxy-äthylhexanoat | 65° /0,001 | 1,4365 |
| 6-Hydroxy-propylhexanoat | 80° /0,09 | 1,4400 |
| 6-Hydroxy-isopropylhexanoat | 75° /0,06 | 1,4462 |
| 6-Hydroxy-butylhexanoat | 85° /0,05 | 1,4412 |
| 6-Hydroxy-isobutylhexanoat | 92° /0,2 | 1,4396 |
| 6-Hydroxy-allylhexanoat | 98° /0,25 | 1,4535 |
| 6-Hydroxy-methallylhexanoat | 99° /0,05 | 1,4580 |

Die neuen Produkte I und ihre Eigenschaften sind in der untenstehenden Tabelle A zusammengestellt.

(Siehe Tabelle A Seite 5 ff.)

Tabelle A

| Ausgangsmaterial II | Reagens | Produkt I | | Sdp (mmHg) | $n_D^{20}$ | $d_4^{20}$ | Geruch |
|---|---|---|---|---|---|---|---|
| $R^1$ | | $R^2$ | $R^1$ | | | | |
| $-CH_2CH_3$ | Acetanhydrid/ Phosphorsäure | $CH_3-$ | $-CH_2CH_3$ | 65/0,1 | 1,4292 | 1,0081 | fruchtig (Himbeeren) |
| $-CH_3$ | Acetanhydrid/ Ameisensäure | H | $-CH_3$ | 107/7 | 1,4310 | 1,0545 | fruchtig, nach Schokolade, Iris |
| $-CH_3$ | Acetanhydrid/ Phosphorsäure | $CH_3-$ | $-CH_3$ | 58/0,08 | 1,4285 | 1,0327 | fruchtig (Himbeeren), blumig |
| $-CH_3$ | Propionsäureanhydrid/Phosphorsäure | $CH_3CH_2-$ | $-CH_3$ | 122/7 | 1,4318 | 1,0148 | fruchtig (Himbeeren, Erdbeeren), Narzisse |

(Fortsetzung)

| | | | | | | | |
|---|---|---|---|---|---|---|---|
| $-CH_2CH_3$ | Acetanhydrid/ Ameisensäure | H | $-CH_2CH_3$ | 47/0,04 | 1,4302 | | fruchtig (Himbeeren) |
| $-CH_2CH_3$ | Propionsäureanhydrid/Phosphorsäure | $CH_3CH_2-$ | $-CH_2CH_3$ | 114/7 | 1,4304 | 0,9937 | fruchtig (Erdbeeren, Himbeeren) |
| $-CH_2CH_2CH_3$ | Acetanhydrid/ Ameisensäure | H | $-CH_2CH_2CH_3$ | 52/0,03 | 1,4323 | 1,0104 | schwach fruchtig |
| $-CH_2CH_2CH_3$ | Acetanhydrid/ Phosphorsäure | $CH_3-$ | $-CH_2CH_2CH_3$ | 66/0,05 | 1,4310 | 0,9928 | schwach fruchtig |
| $-CH_2CH_2CH_3$ | Propionsäureanhydrid/Phosphorsäure | $CH_3CH_2$ | $-CH_2CH_2CH_3$ | 85,5/0,22 | 1,4335 | | schwach fruchtig (Himbeeren) pudrig |

(Fortsetzung)

| | | | | | | | |
|---|---|---|---|---|---|---|---|
| 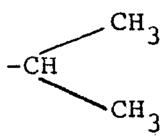 | Acetanhydrid/ Ameisensäure | H | 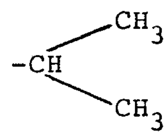 | 68,5/0,2 | 1,4288 | 1,0011 | fruchtig (Ananas),blumig |
| 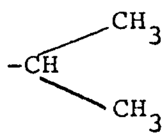 | Acetanhydrid/ Phosphorsäure | $CH_3$- | 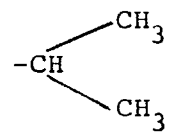 | 78,5/0,2 | 1,4270 | 0,9847 | fruchtig (Himbeeren), pudrig |
| 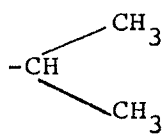 | Propionsäureanhydrid/Phosphorsäure | $CH_3CH_2$- | 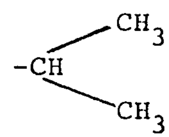 | 67/0,01 | 1,4290 | 0,9733 | fruchtig, nach Birke, Leder |
| $-CH_2CH_2CH_2CH_3$ | Acetanhydrid/ Phosphorsäure | $CH_3$- | $-CH_2CH_2CH_2CH_3$ | 86/0,09 | 1,4330 | 0,9812 | schwach aldehydisch |
| 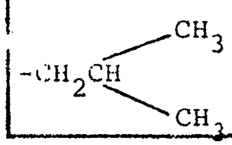 | Acetanhydrid/ Ameisensäure | H | 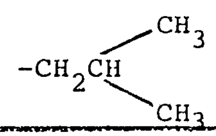 | 66/0,11 | 1,4309 | 0,9910 | nach Hexylsalicylat |

(Fortsetzung)

| | | | | | | | |
|---|---|---|---|---|---|---|---|
| 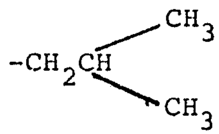 | Acetanhydrid/ Phosphorsäure | $CH_3-$ | 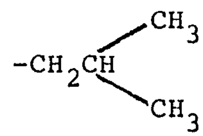 | 74/0,15 | 1,4307 | 0,9765 | etwas blumig, nach Nerolidol |
| $-CH_2CH=CH_2$ | Acetanhydrid/ Ameisensäure | H | $-CH_2CH=CH_2$ | 69/0,1 | 1,4434 | 1,0344 | fruchtig, allylisch |
| $-CH_2CH=CH_2$ | Acetanhydrid/ Phosphorsäure | $CH_3-$ | $-CH_2CH=CH_2$ | 94/0,09 | 1,4430 | 1,0158 | fruchtig, |
| $-CH_2CH=CH_2$ | Propionsäurean-hydrid/Phosphor-säure | $CH_3CH_2-$ | $-CH_2CH=CH_2$ | 87/0,15 | 1,4423 | 1,0014 | fruchtig (Him-beeren), gut haftend |
| $-CH_2CH_3$ | Isobuttersäure-anhydrid/Phos-phorsäure | 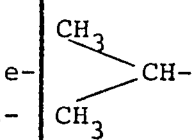 | $CH_2CH_3$ | 72/0,1 | 1,4305 | 0,9748 | fruchtig (Him-beeren) |

Beispiel 2

In einem mit Tropftrichter, Thermometer und Rührer versehenen Rundkolben werden 160 g 6-Hydroxy-äthylhexanoat, 145,4 g N,N-Dimethylanilin und 200 ml wasserfreies Toluol vorgelegt. Innerhalb einer halben Stunde werden unter Rühren 119,4 g Chlorameisensäureäthylester bei 25 °C zugetropft. Das Reaktionsgemisch wird während 3 Stunden unter Rückfluss gehalten. Nach dem Abkühlen giesst man auf Eiswasser, wäscht die organische Phase zweimal mit Wasser, mit 100 ml einer 5 %-igen Salzsäurelösung und hierauf noch mit Wasser neutral. Nach dem Trocknen über Natriumsulfat wird das Lösungsmittel abdestilliert. Man erhält 173 g Rohprodukt. Die fraktionierte Destillation ergibt 112 g (Ausbeute 48,3 % der Theorie) chemisch reines 6-Aethoxycarbonyloxy-äthylhexanoat, davon können 75 g ohne weitere Behandlung als olfaktisch reines Produkt verwendet werden.

Auf analoge Weise werden ausgehend aus den Hydroxyestern $HO(CH_2)_5COOR^1$ (II) die weiteren Alkoxycarbonyloxy-hexanoate $R^2COO(CH_2)_5COOR^1$ (I) mit $R^2$ = Methoxy und Aethoxy hergestellt.

Die neuen Produkte und ihre Eigenschaften sind in der untenstehenden Tabelle B zusammengestellt.

(Siehe Tabelle B Seite 10 ff.)

Tabelle B

| Ausgangs-material II | Reagens | Produkt I | | Sdp[$^{o}C$/mmHg] | $n_D^{20}$ | Geruch |
|---|---|---|---|---|---|---|
| $R^1$ | | $R^2$ | $R^1$ | | | |
| $-CH_2CH_3$ | $ClCOOCH_2CH_3$ | $CH_3CH_2O-$ | $-CH_2CH_3$ | $102^{o}$/0,5 | 1,4300 | fruchtig, an Himbeer-keton erinnernd |
| $-CH_2CH_3$ | $ClCOOCH_3$ | $CH_3O-$ | $-CH_2CH_3$ | $70^{o}$/0,02 | 1,4293 | leicht fruchtig, nach Himbeere |
| $-CH_3$ | $ClCOOCH_2CH_3$ | $CH_3CH_2O-$ | $-CH_3$ | $68^{o}$/0,05 | 1,4292 | fruchtig, nach Him-beere, ölig |
| $-CH_3$ | $ClCOOCH_3$ | $CH_3O-$ | $-CH_3$ | $63^{o}$/0,02 | 1,4290 | fruchtig, nach Verdyl-acetat, leicht nach grünen Aepfeln, ölig |
| $-CH_2CH_2CH_3$ | $ClCOOCH_2CH_3$ | $CH_3CH_2O-$ | $-CH_2CH_2CH_3$ | $88^{o}$/0,07 | 1,4310 | leicht nach Himbeer, ölig |

(Fortsetzung)

| | | | | | |
|---|---|---|---|---|---|
| $-CH_2CH_2CH_3$ | $ClCOOCH_3$ | $CH_3O-$ $-CH_2CH_2CH_3$ | $78^{o}/0,09$ | 1,4308 | ölig, metallisch |
| $-CH_2-CH=CH_2$ | $ClCOOCH_2CH_3$ | $CH_3CH_2O-$ $-CH_2-CH=CH_2$ | $96^{o}/0,15$ | 1,4410 | nach Himbeerkonfitüre |

Beispiel 3

A. Fruchtige Base Richtung Apfel

| | Gewichtsteile |
|---|---|
| Galaxolid 50 (IFF) (1,3,4,6,7,8-Hexahydro-4,6,6,7,8,8-hexamethyl-cyclopenta-γ-2-benzopyran) in Diäthylphthalat | 100 |
| Fraise pure (Aethyl-methyl-phenylglycidat) [50 % in Aethylphthalat] | 100 |
| Agrumex (Haarmann & Reimer) (2-tert.-Butylcyclohexyl-acetat) | 60 |
| Maltylisobutyrat | 30 |
| cis-3-Hexenylisobutyrat | 60 |
| Dimethylbenzylcarbinylbutyrat | 40 |
| Givescone (Giv) (2-Aethyl-6,6-dimethyl-2-cyclohexen-1-carbonsäureäthylester) | 20 |
| Cyclal C (Giv) (1,3-Dimethyl-cyclohex-1-en 4 (und 5)-carboxaldehyd) (10 % DPG) | 10 |
| Acetessigester | 40 |
| Dipropylenglycol (DPG) | 440 |
| | 900 |

Gibt man zu obiger, allgemein fruchtigen Base 100 Teile 6-Acetoxy-äthyl-hexanoat, wird die fruchtige Note sehr deutlich in Richtung Apfel gebracht. Die pudrig-fruchtige Note des Galaxolid-Agrumex Komplexes wird zudem in vorteilhafter Weise unterstrichen, was der Base einen sehr natürlichen und gut haftenden Charakter verleiht.

B. Fruchtige Base Richtung Melone

| | Gewichtsteile |
|---|---|
| Myraldylacetat (Giv) (5-[3- und 4-(Acetoxymethyl)-1-cyclohexenyl]-2-methyl-2-penten) | 100 |
| Linalylacetat | 100 |
| Dimethylbenzylcarbinylbutyrat | 50 |
| Cyclamenaldehyd | 30 |
| Fraise pure (Aethyl-methyl-phenyl-glycidat) | 30 |
| Undecalacton | 30 |
| Geranylacetat | 30 |
| Acetessigester | 30 |
| Acetal R (Giv) (Acetaldehyd-phenyl-äthyl-n-propylacetal) | 10 |
| Melonia (3,7-Dimethyl-7-methoxy-1-octanal) | 5 |
| Dipropylenglykol (DPG) | 350 |
| | 765 |

Durch Zusatz von 100 Teilen 6-Acetoxy-äthyl-hexanoat wird obige Base von allgemeinem fruchtigem Charakter in Richtung Zuckermelone gebracht. Die lactonartige, süsse Note wird deutlich verstärkt, was der Base mehr Volumen und wesentlich bessere Haftfestigkeit verleiht.

C. Parfumerie Base Richtung Aprikose

| | Gewichtsteile |
|---|---|
| α-Ionon | 80 |
| Dimethylbenzylcarbinylbutyrat | 50 |
| Allylionon | 40 |
| Fructone R (IFF) (2-Methyl-1,3-dioxolan-2-äthylacetat) | 30 |
| Palmarosaöl | 20 |
| Undecalakton | 15 |
| Dipropylenglykol (DPG) | 665 |
| | 900 |

Ein Zusatz von 100 Teilen-6-Acetoxy-äthyl-hexanoat erbringt in obiger konventionellen Aprikosenbase die typische, ausgesprochen samtig-weiche Note der überreifen Aprikose ; die entstandene Formulierung eignet sich bereits gut zur Parfumierung von kosmetischen Präparaten ; mit anderen Worten : die nun entstandene Base ist nun eine echte, vollständige Parfumbase. Im Falle der konventionnellen Aprikosenbase ist dies noch nicht der Fall : Zwar kann auch sie in einer Komposition eine Aprikosennuance erbringen, doch fehlt der Komposition in der Folge die süsse, samtige Note der reifen Frucht.

D. Blumig-grüne Base

| | Gewichtsteile |
|---|---|
| Phenoxyäthylalkohol | 205 |
| Linalool | 150 |
| Terpineol | 100 |
| Dimethylbenzylcarbinylbutyrat | 60 |
| Galaxolid (IFF) (1,3,4,6,7,8-Hexahydro-4,6,6,7,8,8-hexamethyl-cyclopenta-γ-2-benzopyran) | 60 |
| Hedione (Firmenich (Methyldihydrojasmonat) | 60 |
| Verdylacetat (Dihydro-nor-dicyclopentadienyl-acetat) | 50 |
| Citronellol | 40 |
| Acetal R (Giv) (Acetaldehyd-phenyläthyl-n-propylacetal) | 30 |
| Geranylacetat | 30 |
| Lilial (Giv) (p-tert.Butyl-α-methylhydrozimtaldehyd) | 15 |
| Cyclamenaldehyd (10 % DPG) | 10 |
| Cyclal C (Giv) (1,3-Dimethyl-cyclohex-1-en 4(und 5)-carboxaldehyd) (10 % DPG) | 10 |
| Phenylacetaldehyddimethylacetal | 10 |
| Corps Cassis (Giv) (p-Menthan-8-thiol-3-on (10 % DPG) | 10 |
| Eugenol | 10 |
| Diphenyloxyd | 5 |
| Indol (10 % DPG) | 5 |
| Dipropylenglykol (DPG) | 40 |
| | 900 |

Gibt man zu obiger blumig-grünen Base 100 Teile 6-Acetoxy-äthyl-hexanoat, entsteht eine sehr viel grünere, pudrigere und wesentlich geruchsstärkere Base. Der neue Ester verbindet die Moschus- und die grüne Noten (Galaxolid-Acetal R) sehr vorteilhaft miteinander. Die Base eignet sich sehr gut zur Parfumierung von kosmetischen Präparaten.

E. Krautig-grüne Base

| | Gewichtsteile |
|---|---|
| Linalylacetat | 150 |
| Madrox (Giv) (1-Methyl-1-methoxy-cyclododecan) | 100 |
| Linalool | 150 |
| Tetrahydrolinalool | 80 |
| Hedione (Firmenich) (Methyl-dihydrojasmonat) | 100 |
| Bergamotteöl | 80 |
| Ysopöl | 60 |
| Patchouliöl (Anhydrol) | 60 |
| Galaxolid (IFF) (1,3,4,6,7,8-Hexahydro-4,6,6,7,8,8-hexamethyl-cyclopenta-α-2-benzopyran) | 40 |
| Allylionon | 20 |
| Eugenylphenylacetat | 20 |
| Acetal E (Giv) (1-Phenyl-4-methyl-3,5-dioxyheptan) | 15 |
| Cyclal C (Giv) (1,3-Dimethyl-cyclohex-1-en 4(und 5)-carboxaldehyd) (10 % DPG) | 10 |
| Corps cassis (Giv) (p-Menthan-8-thio-3-on) (10 % DPG) | 10 |
| Fixateur 404 (Firmenich) (8-α,12-Oxido-13,14,15,16-tetranorlabdan) | 5 |
| | 900 |

Durch Zusatz von 10 % 6-Acetoxy-äthyl-hexanoat wird die krautig-grüne Note obiger Base eindeutig verstärkt. 6-Acetoxy-äthyl-hexanoat unterstreicht in ungeahnter Weise die etwas pudrige, würzige Note der Grundkomposition, was der Base viel Wärme und Volumen bringt. Man kann die neue Formulierung nun sehr gut für Herren-Colognes verwenden.

In keiner einzigen der obigen Basen A bis E können hingegen durch Ersatz von 6-Acetoxy-äthyl-hexanoat mittels derselben Mengen der strukturell ähnlichen Verbindungen Diäthyladipat oder 1,6-Diacetoxyhexan die aufgeführten Effekte erzielt werden.

Beispiel 4

A. Krautig-grüne Base

| | Gewichtsteile |
|---|---|
| Fixateur 404 | 5 |
| Corps Cassis DC (10 % DPG) | 10 |

| | Gewichtsteile |
|---|---|
| Cyclal C (10 % DPG) | 10 |
| Acetal E | 15 |
| Eugenyl-phenylacetat | 20 |
| Keton V (Allyl-α-ionon) | 20 |
| Galaxolid 50 | 40 |
| Patchouli-Anhydrol | 60 |
| Ysopöl | 60 |
| Bergamotteöl | 80 |
| Tetrahydrolinalool | 80 |
| Madrox | 100 |
| Hedione | 100 |
| Linalool | 150 |
| Linalylacetat | 150 |
| | 900 |

Der Zusatz von 100 Teilen 6-Aethoxycarbonyloxy-äthyl-hexanoat verstärkt den fruchtig-hesperidenartigen Aspekt der Base und verleiht der neuen Komposition bemerkenswerte Frische. Im Vergleich zu der Komposition ohne Zusatz wird die neue Komposition ganz eindeutig vorgezogen.

B. Fruchtige Base

| | Gewichtsteile |
|---|---|
| Cyclal C (10 % DPG) | 10 |
| Givescone | 20 |
| Maltylisobutyrat | 30 |
| Dimethylbenzylcarbinylbutyrat | 40 |
| Aethylacetat | 40 |
| cis-3-Hexenylisobutyrat | 60 |
| Agrumex | 60 |
| Galaxolid 50 | 100 |
| Fraise pure | 100 |
| Dipropylenglycol (DPG) | 440 |
| | 900 |

Ein Zusatz von 100 Teilen 6-Aethoxycarbonyloxy-äthyl-hexanoat führt zu einer eindeutigen Bevorzugung der so hergestellten neuen Komposition, da sich in ihr der erwünschte Effekt in Richtung Apfel wesentlich deutlicher zu erkennen gibt als in der Grundbase ohne Zusatz. Eine erwünschte Typisierung einer primär allgemein-fruchtigen Note wird somit durch den Zusatz der neuen Verbindung I bewirkt, wobei der Erdbeeraspekt (« Fraise pure ») in eine Apfelnote übergeht. Dieser Effekt ist überdies unerwartet, da die neue Verbindung I fruchtig in Richtung Himbeere riecht, und man somit vielmehr eine Verstärkung der fruchtig-beerenartigen Note erwarten würde.

Base in Richtung Melone

| | Gewichtsteile |
|---|---|
| Melonia | 5 |
| Acetal R | 10 |
| Aethylacetat | 30 |
| Geranylacetat | 30 |
| Pêche pure (γ-n-heptylbutyrolacton) | 30 |
| Fraise pure | 30 |
| Cyclamenaldehyd | 30 |
| Dimethylbenzylcarbinylbutyrat | 50 |
| Myraldylacetat | 100 |
| Linalylacetat | 100 |
| Dipropylenglykol (DPG) | 350 |
| | 765 |

Der Zusatz von 100 Teilen 6-Aethoxycarbonyloxy-äthyl-hexanoat zur der Grundbase wirkt sich organoleptisch ausserordentlich günstig aus, da der fruchtig-saftige Melonencharakter hierdurch bedeutend akzentuiert wird.

**Patentansprüche**

1. Verbindungen der Formel

$$R^2COO—(CH_2)_5—COOR^1 \quad (I)$$

worin $R^1$ $C_{1-4}$-Alkyl oder $C_{2-4}$-Alkenyl und $R^2$ Wasserstoff, $C_{1-3}$-Alkyl, $C_{2-3}$-Alkenyl oder $C_{1-2}$-Alkoxy bedeuten.

2. 6-Acetoxy-äthyl-hexanoat.

3. 6-Isobutyroxy-äthyl-hexanoat.

4. 6-Propionoxy-allyl-hexanoat.

5. 6-Aethoxycarbonyloxyäthylhexanoat.

6. Riechstoffkomposition, gekennzeichnet durch einen Gehalt an einer Verbindung der Formel

$$R^2COO—(CH_2)_5—COOR^1 \quad (I)$$

worin $R^1$ $C_{1-4}$-Alkyl oder $C_{2-4}$-Alkenyl und $R^2$ Wasserstoff, $C_{1-3}$-Alkyl, $C_{2-3}$-Alkenyl oder $C_{1-2}$-Alkoxy bedeuten.

7. Verfahren zur Herstellung von Verbindungen der Formel

$$R^2COO—(CH_2)_5—COOR^1 \quad (I)$$

worin $R^1C_{1-4}$-Alkyl oder $C_{2-4}$-Alkenyl und $R^2$ Wasserstoff, $C_{1-3}$-Alkyl, $C_{2-3}$-Alkenyl oder $C_{1-2}$-Alkoxy bedeuten,
dadurch gekennzeichnet, dass man eine Verbindung der Formel

$$HO—(CH_2)_5—COOR^1 \quad (II)$$

worin $R^1$ obige Bedeutung hat, verestert.

8. Verwendung von Verbindungen der Formel

$$R^2COO—(CH_2)_5—COOR^1 \quad (I)$$

worin $R^1$ $C_{1-4}$-Alkyl oder $C_{2-4}$-Alkenyl und $R^2$ Wasserstoff, $C_{1-3}$-Alkyl, $C_{2-3}$-Alkenyl oder $C_{1-2}$-Alkoxy bedeuten, als Riechstoffe.

**Claims**

1. Compounds of the formula

$$R^2COO—(CH_2)_5—COOR^1 \quad (I)$$

wherein $R^1$ signifies $C_{1-4}$-alkyl or $C_{2-4}$-alkenyl and $R^2$ signifies hydrogen, $C_{1-3}$-alkyl, $C_{2-3}$-alkenyl or $C_{1-2}$-alkoxy.

2. Ethyl 6-acetoxy-hexanoate.

3. Ethyl 6-isobutyroxy-hexanoate.

4. Allyl 6-propionoxy-hexanoate.

5. Ethyl 6-ethoxycarbonyloxyhexanoate.

6. An odorant composition, characterized by a content of a compound of the formula

$$R^2COO—(CH_2)_5—COOR^1 \quad (I)$$

wherein $R^1$ sifgnifies $C_{1-4}$-alkyl or $C_{2-4}$-alkenyl and $R^2$ signifies hydrogen, $C_{1-3}$-alkyl, $C_{2-3}$-alkenyl or $C_{1-2}$-alkoxy.

7. A process for the manufacture of compounds of the formula

$$R^2COO—(CH_2)_5—COOR^1 \quad (I)$$

wherein $R^1$ signifies $C_{1-4}$-alkyl or $C_{2-4}$-alkenyl and $R^2$ signifies hydrogen, $C_{1-3}$-alkyl, $C_{2-3}$-alkenyl or $C_{1-2}$-alkoxy,
characterized by esterifying a compound of the formula

$$HO—(CH_2)_5—COOR^1 \quad (II)$$

wherein $R^1$ has the above significance.

8. The use of compounds of the formula

$$R^2COO—(CH_2)_5—COOR^1 \quad (I)$$

wherein $R^1$ signifies $C_{1-4}$-alkyl or $C_{2-4}$-alkenyl and $R^2$ signifies hydrogen, $C_{1-3}$-alkyl, $C_{2-3}$-alkenyl or $C_{1-2}$-alkoxy, as odorants.

**Revendications**

1. Composés de formule

$$R^2COO—(CH_2)_5—COOR^1 \quad (I)$$

dans laquelle $R^1$ représente un groupe alkyle en $C_1$-$C_4$ ou alcényle en $C_2$-$C_4$ et $R^2$ représente l'hydrogène, un groupe alkyle en $C_1$-$C_3$, alcényle en $C_2$-$C_3$ ou alcoxy en $C_1$-$C_2$.

2. Le 6-acétoxy-hexanoate d'éthyle.

3. Le 6-isobutyroxy-hexanoate d'éthyle.

4. Le 6-propionoxy-hexanoate d'allyle.

5. Le 6-éthoxycarbonyloxyhexanoate d'éthyle.

6. Composition odorante, caractérisée en ce qu'elle contient un composé de formule

$$R^2COO—(CH_2)_5—COOR^1 \quad (I)$$

dans laquelle $R^1$ représente un groupe alkyle en $C_1$-$C_4$ ou alcényle en $C_2$-$C_4$ et $R^2$ représente l'hydrogène, un groupe alkyle en $C_1$-$C_3$, alcényle en $C_2$-$C_3$ ou alcoxy en $C_1$-$C_2$.

7. Procédé de préparation des composés de formule

$$R^2COO—(CH_2)_5—COOR^1 \quad (I)$$

dans laquelle $R^1$ représente un groupe alkyle en $C_1$-$C_4$ ou alcényle en $C_2$-$C_4$ et $R^2$ représente l'hydrogène, un groupe alkyle en $C_1$-$C_3$, alcényle en $C_2$-$C_3$ ou alcoxy en $C_1$-$C_2$, caractérisé en ce que l'on estérifie un composé de formule

$$HO—(CH_2)_5—COOR^1 \quad (II)$$

dans laquelle $R^1$ a les significations indiquées ci-dessus.

8. Utilisation des composés de formule

$$R^2COO—(CH_2)_5—COOR^1 \quad (I)$$

dans laquelle $R^1$ représente un groupe alkyle pen $C_1$-$C_4$ ou alcényle en $C_2$-$C_4$ et $R^2$ représente l'hydrogène, un groupe alkyle en $C_1$-$C_3$, alcényle en $C_2$-$C_3$ ou alcoxy en $C_1$-$C_2$, en tant que matières odorantes.